# EUROPEAN PATENT SPECIFICATION

(11) **EP 2 609 894 B1**
(45) Date of publication and mention of the grant of the patent: **01.04.2015**
(21) Application number: 12154524.8
(22) Date of filing: 08.02.2012
(51) Int. Cl.: A61F 2/24

(54) **Heart valve**
Herzklappe
Valve cardiaque

(30) Priority: 31.12.2011 PL 39762511; 31.12.2011 EP 11196280
(43) Date of publication of application: 03.07.2013
(73) Proprietor: Fundacja Rozwoju Kardiochirurgii Im. Prof. Zbigniewa Religi, 41-800 Zabrze (PL)
(72) Inventor: Józwik, Krzysztof, 92-434 Lódz (PL); Witkowski, Dariusz, 87-820 Kowal (PL); Obidowski, Damian, 95-020 Stróza (PL); Moll, Jacek, 90-415 Lódz (PL); Kustosz, Roman, 41-800 Zabrze (PL); Szuber, Agnieszka, 44-121 Gliwice (PL); Kapis, Artur, 41-800 Zabrze (PL)
(74) Representative: Malcherek, Piotr

(56) References cited:
- GB-A- 1 292 320
- US-A- 4 240 161

## Description

The subject of the invention is a heart valve, in particular an artificial disc heart valve designed to be situated in the outlet channel of a ventricular heart assist device.

There are various structures of artificial disc heart valves. For example, an American patent description with the publication number US 4240161 discloses an artificial disc heart valve comprising a tilting disc mounted on a heart valve ring by means of two pairs of holders, symmetrical to the plane of symmetry of the heart valve ring. Each pair of said means comprises a lower holder and an upper holder and the disc in the closed position of the heart valve is supported by an additional protrusion on the inner surface of the heart valve ring which protrusion confines the disc in the extreme position of the heart valve disc and is located within the plane of symmetry of the heart valve. In different embodiments of said invention it was disclosed that the upper surface of the heart valve disc may be convex and the lower surface of the heart valve disc may be concave.

Furthermore, there are other American patent descriptions with publication numbers US 4725275 and US 4661106 which disclose other structures of artificial disc heart valves.

The present invention relates to a heart valve comprising a tilting disc mounted on a heart valve ring by means of two pairs of holders symmetrical to the plane of symmetry of the heart valve ring, wherein each pair of said holders comprises a lower holder and an upper holder and the disc in the closed position of the heart valve is supported by an additional protrusion on the inner surface of the heart valve ring which is a limiter of the extreme position of the heart valve disc and is located within the plane of symmetry of the heart valve. The upper surface of the heart valve disc is convex and the lower surface of the heart valve disc is concave. The essence of the invention is that each of the lower holder for supporting the heart valve disc has at its end a spherical surface and they are so arranged that in the projection on a surface parallel to the longitudinal axis of the heart valve the central points of the spherical surfaces of the lower holders and the point defined by the projection of a line defined by the plane of symmetry of the heart valve and an outline of the cylindrical inner surface of the heart valve ring form an equilateral triangle whose sides run from point X to the projections of the central points of the spherical surfaces. The angle between the sides of said equilateral triangle is within the range of over 53° and less than 60°. The heart valve disc around the rim thereof has a circumferential spline whose inner wall that leans against the spherical surfaces of the lower holders is inclined in relation to the axis of symmetry of the heart valve disc, wherein the angle of inclination is within the range between 0.5 and 0.62 of the angle of mutual inclination of the sides of the equilateral triangle referred to above.

In a preferred embodiment in the assembled state of the heart valve disc the distance between the upper holder and the lower holder in each pair of holders is within the range between 0.048 - 0.1 of the diameter of the heart valve disc.

It is also preferable that the thickness of the disc including the circumferential spline is within the range between 0.06 - 0.12 of the diameter D of the heart valve disc.

Moreover, it is advisable that the upper surface of the disc is a spherical surface with the same radius of curvature as the lower spherical surface of the disc and the working surface of the upper holder is tangent to the curvature of the upper spherical surface of the disc in the point where the upper holder touches the surface of the heart valve disc, in each position of the heart valve disc.

It is also preferable that the lower holders, the upper holders and the limiter of the extreme position of the heart valve disc are located inside the clearance of the heart valve ring and do not extend beyond the upper and lower inner rim of the heart valve ring.

It is also recommendable that the length of each upper holder and of each lower holder and of the limiter of the extreme position of the heart valve disc is less than 0.2 of the diameter of the heart valve.

The upper holder is understood as the holder situated at the inlet side of the heart valve, which means the side where blood flows into the heart valve. The upper, convex surface of the heart valve disc is the disc surface situated at the side where blood flows into the heart valve. Analogically, the lower holder and the lower concave surface of the heart valve disc are situated at the outlet of the heart valve, thus they are at the side where blood flows out from the heart valve. Whereas the length of the upper holder, the length of the lower holder and the length of the limiter is understood as the length of said elements in the projection on the plane perpendicular to the axis of symmetry of the heart valve ring.

The heart valve according to the invention is a low-profile heart valve, in which, owing to the new position of the holders confining the heart valve position to the outline of the heart valve ring and the structure thereof, the undesirable vibration of the heart valve disc was eliminated. In particular, the described position of the means for holding and confining the heart valve disc allows to achieve the turning moment affecting the heart valve disc resulting from the pressure of the blood surrounding the disc and simultaneously prevents self-induced vibrations of the disc end at the heart valve outlet. What is more, the structure of the heart valve allows for optimum characteristics of blood flow through the heart valve, while minimising pressure gradients and stagnation areas where the risk of thrombosis is increased and while reducing to the maximum the forced prewhirl of blood. By mutual selection of an angle between the sides of the equilateral triangle described above and the angle of inclination of the inner surface of the circumferential spline around the lower concave surface of the heart valve disc the desired matching of the cooperating surfaces of the holders and the heart valve disc is achieved. The shape of the supporting holders is fully matched with the outline of the heart valve disc. Such geometry prevents coming out of the disc having a circumferential spline during operation of the heart valve. Prior to mounting the disc on the heart valve ring, the distance between the upper and lower holders allows to place the disc in the appropriate position easily. After that the upper holders are bent to the position preventing the possibility of coming out of the disc and at the same time allows the disc to take extreme positions in the closed position and the fully open position.

The invention is presented in greater detail in the following embodiments and in the attached schematic drawing where fig.1 is a perspective top view of the heart valve ring, thus from the side of the blood inlet prior to mounting the heart valve disc within, fig. 2 is a perspective bottom view of the heart valve ring, thus from the side of the blood outlet prior to mounting the heart valve disc within, fig. 3 is a top view of the heart valve ring prior to mounting the heart valve disc within, fig. 4 is a bottom view of the heart valve ring prior to mounting the heart valve disc within, fig. 5 is a bottom view of the heart valve in the open position of the disc, fig. 6 is a longitudinal section of the heart valve in the open position of the disc, fig. 7 is a longitudinal section of the heart valve ring prior to bending the upper holders, fig. 8 is a longitudinal section of the heart valve ring after bending the upper holders with no heart valve disc visible, fig. 9 is a longitudinal section of the heart valve with the disc visible in the closed position, fig. 10a and 10b are schematic views of the upper holder and lower holder before and after bending, fig. 11 is a partial sectional view of the heart valve disc, fig. 12 is a projection of the heart valve ring on the plane perpendicular to the axis of symmetry of the heart valve ring, and fig. 13 is a partial schematic section of the heart valve ring and disc in the closed position of the disc.

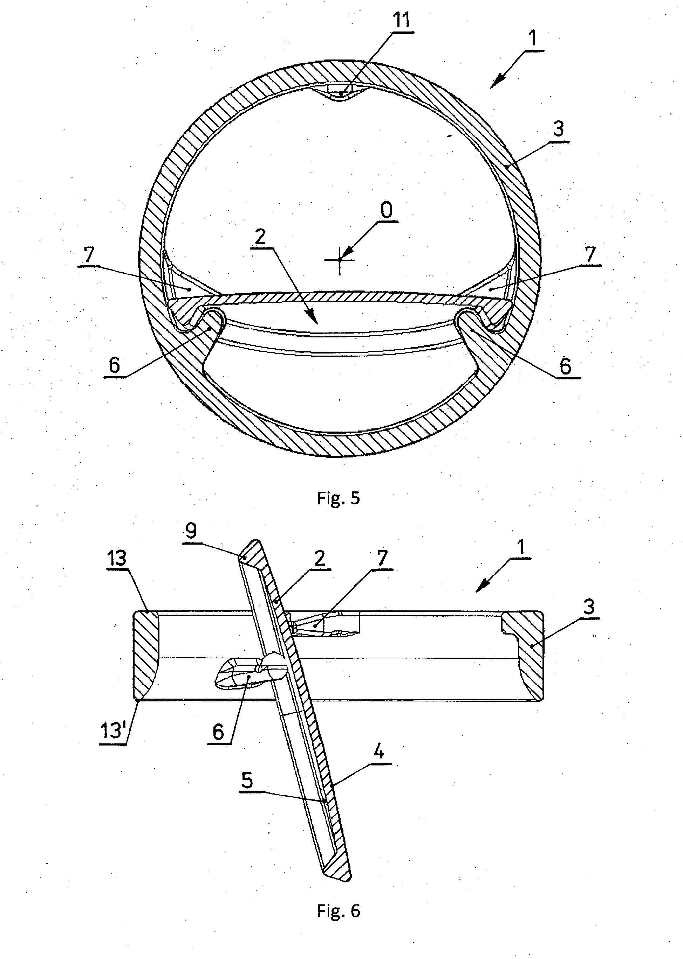

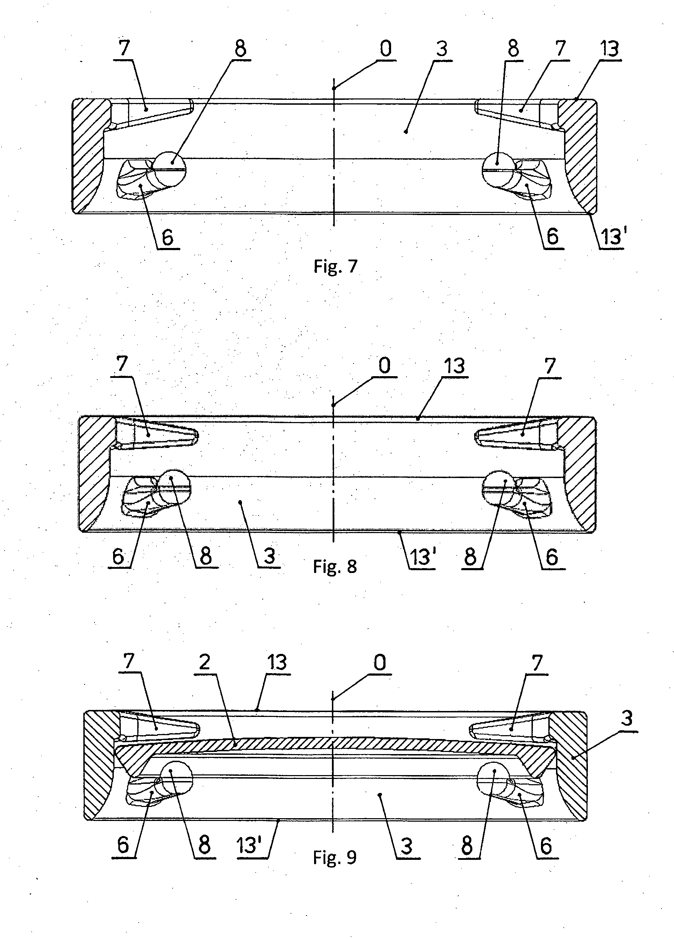

An artificial disc heart valve 1 according to the invention comprises a tilting disc 2 mounted on a ring 3 of the heart valve 1. The disc 2 has a convex upper surface 4 from the side of blood inlet to the heart valve and a concave lower surface 5 from the side of blood outlet from the heart valve ( fig. 6 - fig. 9). Radiuses of the spherical curvature of the upper surface 4 and the lower surface 5 of the disc 2 are the same. The disc 2 is mounted on the ring 3 of the heart valve 1 by means of two pairs of holders, and each pair of holders comprises a lower holder 6 from the side of blood outlet from the heart valve and an upper holder 7 from the side of blood inlet to the heart valve. The pairs of holders 6,7 are arranged symmetrically in relation the symmetry plane P of the ring 3 of the heart valve 1.

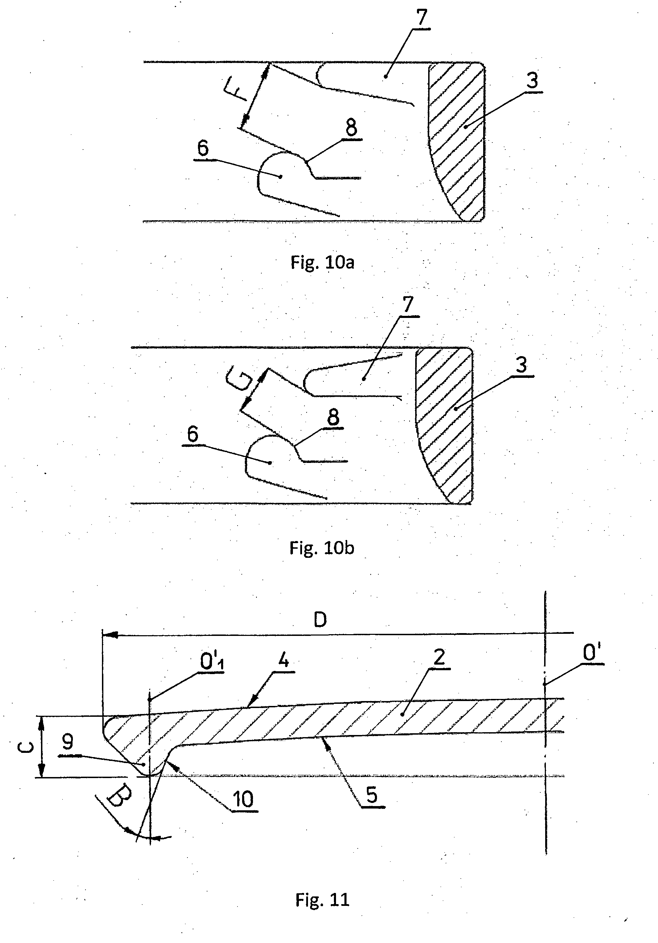

The lower holders 6 supporting the disc 2 of the heart valve have at their ends a spherical surface 8 and are so arranged that in the projection on the plane perpendicular to the longitudinal axis 0 of the heart valve 1 the central points Z of the spherical surfaces 8 of the lower holders 6 and the point X defined by the projection of a line defined by the plane of symmetry P and the heart valve 1 and the outline of the cylindrical inner surface of the ring 3 of the heart valve 1 form an equilateral triangle T with sides S which run from the point X to the projections of the central points Z of the spherical surfaces 8. The angle A between the sides S of said equilateral triangle is within the range of over 53° and less than 60°, depending on the size of the heart valve 1. Around the lower rim thereof the disc 2 has a circumferential spline 9 whose inner wall 10 supporting the disc 2 against the spherical surfaces 8 of the lower holders 6 is inclined at an angle B in relation to the plane of symmetry 0' of the disc 2 of the heart valve 1. Fig. 11 shows the angle B between the wall 10 and the axis 0'₁, which axis 0'₁ is parallel to the axis of symmetry 0' of the disc 2. The inclination angle is within the range between 0.5 and 0.62 of the angle A of mutual inclination of the sides S of the equilateral triangle T depending on the size of the heart valve 1. An example angle A is 53° or 59°, and the angle B - 30° or 31°. Further example angles A and B shall be presented below, in Table 2.

The disc 2 in the closed position of the heart valve 1 is supported by an additional protrusion 11 on the inner surface of the ring 3 of the heart valve 1, which is a limiter of the extreme position of the disc 2. The protrusion 11 is within the plane of symmetry P of the heart valve 1. In the projection of the protrusion 11 on the plane perpendicular to the longitudinal axis 0 of the ring 3 of the heart valve 1 there is a point X which is a peak of the equilateral triangle T.

The lower holders 6 and the upper holders 7 prior to mounting the disc 2 enable mounting said disc 2 between them ( fig. 10a ). Subsequently, the upper holder 7 is bent towards the lower holder 6 in each pair of holders in such a way that the primary distance F between the holders 6, 7 is reduced to distance G ( fig. 10b ). As a result the disc 2 is protected against coming out from the holders 6, 7. The arrangement of the holders 6, 7 allows the disc 2 to open to the maximum angle of 75°.

The distance G in the assembler state of the disc 2 of the heart valve 1 between the upper holder 7 and the lower holder 6 in each pair of holders is within the range of 0.048 - 0.1 of the diameter D of the disc 2 of the heart valve 1, preferably 0.05 - 0.78. The thickness C of the disc 2 with the circumferential spline 9 is within the range of 0.06 - 0.12 of the diameter D of the disc 2 of the heart valve 1, which prevents the possibility of the disc 2 coming out from the holders 6, 7. The upper surface 4 of the disc 2 is a spherical surface with the same radius of curvature as the lower spherical surface 5 of the disc 2 and the working surface of the upper holder 7 is tangent to the curvature of the of the upper spherical surface 4 of the disc 2 in the point K, where the upper holder 7 touches the surface 4 of the disc 2 of the heart valve 1 for each position of the disc 2 of the heart valve 1.

The lower holders 6, the upper holders 7 and the limiter 11 of the extreme position of the disc 2 of the heart valve 1 are located inside the clearance 12 of the ring 3 of the heart valve 1 and do not extend beyond upper and lower edges 13, 13' of the ring 3 of the heart valve 1.

The length of each upper holder 7 and of the lower holders 6 and the limiter 11 of the extreme position of the disc 2 of the heart valve 1 is less than 0.2 of the diameter D of the disc 2 of the heart valve 1. For example, for a heart valve with the disc diameter D=13.9 mm, the length of the lower holder is 2 mm, the length of the upper holder is 2.7 mm, and the length of the limiter is 0.72 mm.

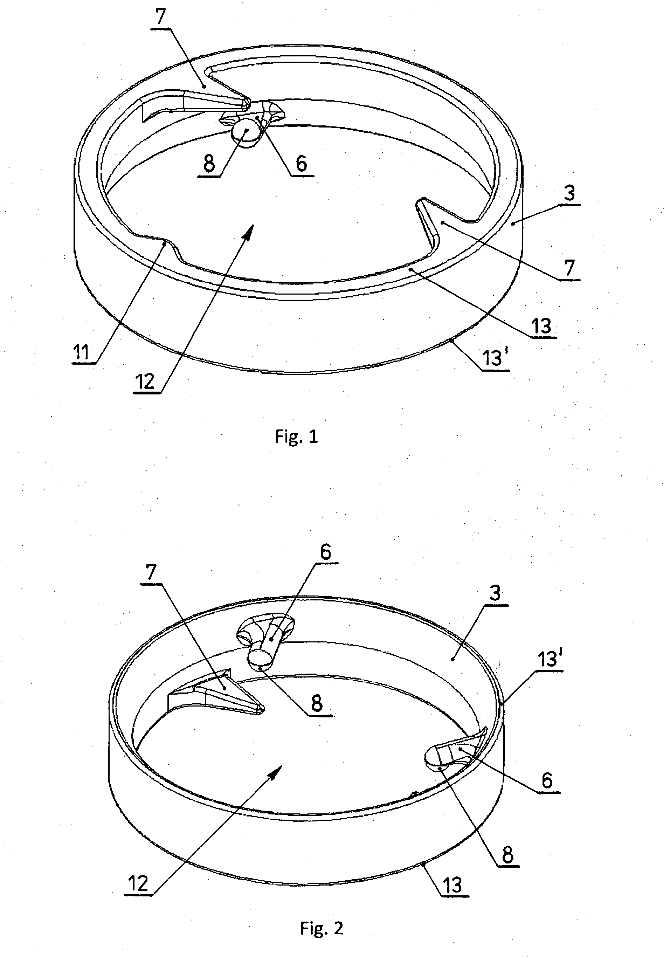

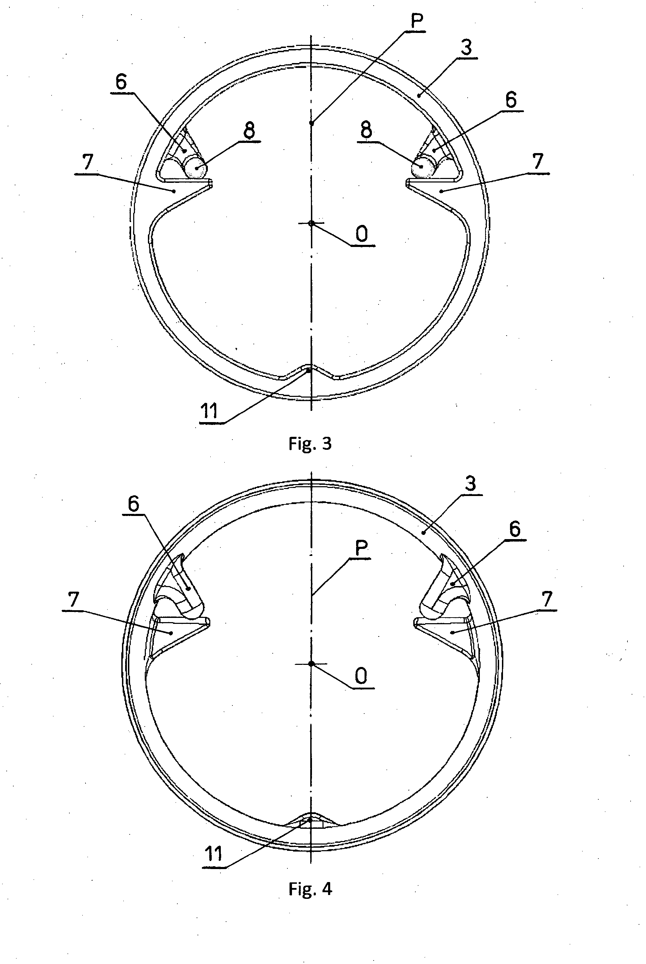

The embodiments described above are shown in greater detail in fig. 1 to fig. 13. Furthermore, the following tables 1 and 2 present example dimensions and dimension ratios for heart valves of different sizes. However, the scope of the invention encompasses embodiments of extreme dimensions and/or ranges and between those dimensions and/or ranges.

**Table 1**

| D [mm] | C [mm] | C/D | R [mm] | E [mm] | R/E | G [mm] | G/D |
|---|---|---|---|---|---|---|---|
| 11.9 | 1.32 | 0.11 | 0.65 | 12 | 0.054 | 1.15 | 0.097 |
| 13.9 | 1.36 | 0.098 | 0.65 | 14 | 0.046 | 1.13 | 0.081 |
| 15.9 | 1.41 | 0.088 | 0.65 | 16 | 0.041 | 1.24 | 0.078 |
| 17.9 | 1.33 | 0.074 | 0.65 | 18 | 0.036 | 1.16 | 0.065 |
| 23.5 | 1.48 | 0.063 | 0.65 | 23.6 | 0.028 | 1.38 | 0.059 |

where
D - diameter of the heart valve disc [ mm ]
C - thickness of the heart valve disc with the lip [ mm ]
R - radius of the spherical surface of the lower holder [ mm ]
E - inner diameter of the heart valve ring [ mm ]
G - distance between the lower holder and the upper holder [ mm ]

**Table 2**

| D [mm] | Angle A [°] | Angle B [°] | Angle B/Angle A |
|---|---|---|---|
| 11.9 | 54 | 33.5 | 0.62 |
| 13.9 | 56.2 | 33.2 | 0.59 |
| 15.9 | 55.2 | 33.1 | 0.60 |
| 17.9 | 55.8 | 33.5 | 0.60 |
| 23.5 | 57.5 | 33.5 | 0.58 |

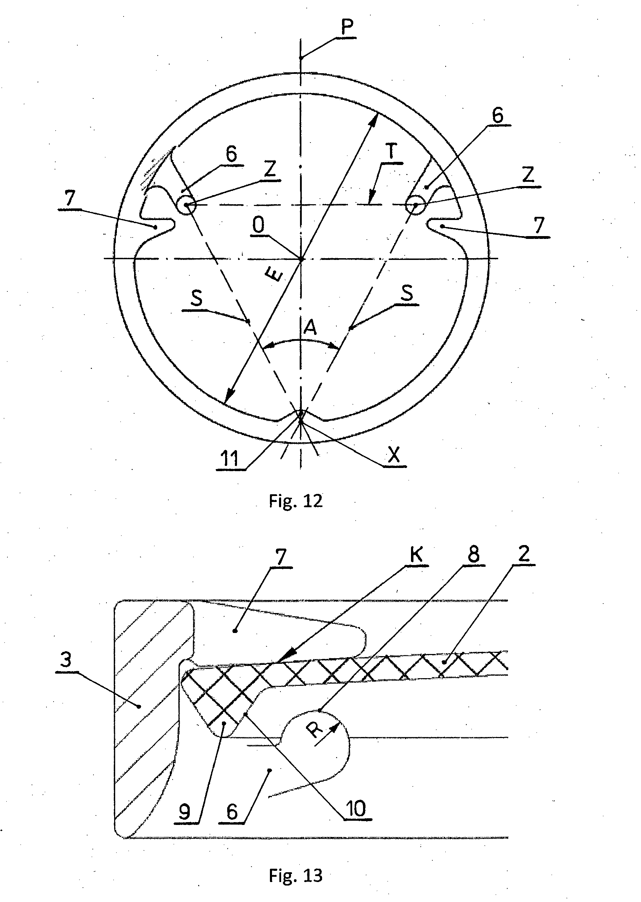

Angle A - the angle between the sides S of the equilateral triangle T (fig. 12)
Angle B - the angle between the wall 10 and the axis of symmetry 0' of the disc 2 (fig. 11)

## Claims

1. A heart valve comprising a tilting disc ( 2 ) mounted on a ring ( 3 ) of the heart valve ( 1 ) by means of two pairs of holders ( 6, 7 ), symmetrical in relation to the plane of symmetry ( P ) of the ring ( 3 ) of the heart valve ( 1 ), wherein each pair of holders ( 6, 7 ) comprises a lower holder ( 6 ) and an upper holder ( 7 ), the disc ( 2 ) in the closed position of the heart valve ( 1 ) is supported by an additional protrusion ( 11 ) on the inner surface of the ring ( 3 ) of the heart valve ( 1 ) constituting a limiter of the extreme position of the disc ( 2 ) of the heart valve ( 1 ) situated in the plane of symmetry ( P ) of the heart valve ( 1 ), whereas the upper surface ( 4 ) of the disc ( 2 ) of the heart valve ( 1 ) is a convex surface, and the lower surface ( 5 ) of the disc ( 2 ) of the heart valve ( 1 ) is concave, **characterised in that** the lower holders ( 6 ) supporting the disc ( 2 ) of the heart valve ( 1 ) have spherical surfaces ( 8 ) at the ends thereof and are so arranged that in the projection on a plane perpendicular to the longitudinal axis ( 0 ) of the heart valve ( 1 ) the central points ( Z ) of the spherical surfaces ( 8 ) of the lower holders ( 6 ) and the point ( X ) defined by the projection of a line defined by the plane of symmetry ( P ) of the heart valve ( 1 ) and an outline of the cylindrical inner surface of the ring ( 3 ) of the heart valve ( 1 ) form an equilateral triangle ( T ) whose sides ( S ) run from the point ( X ) to the projections of the central points ( Z ) of the spherical surfaces ( 8 ), with an angle ( A ) between the sides ( S ) of said equilateral triangle ( T ) within the range over 53° and less than 60°, whereas the disc ( 2 ) of the heart valve ( 1 ) around the rim thereof has a circumferential spline ( 9 ), the inner wall ( 10 ) thereof supporting the disc ( 2 ) towards the spherical surfaces ( 8 ) of the lower holders ( 6 ), is inclined at an angle ( B ) in relation to the axis of symmetry ( 0' ) of the disc ( 2 ) of the heart valve ( 1 ), wherein the angle ( B ) is within the range between 0.5 and 0.62 of the angle ( A ).

2. The heart valve according to claim 1 **characterised in that** the distance ( G ) in the assembled state of the disc ( 2 ) of the heart valve ( 1 ) between the upper holder ( 7 ) and the lower holder ( 6 ) in each pair of holders is within the range of 0.048 - 0.1 of the diameter ( D ) of the disc ( 2 ) of the heart valve ( 1 ).

3. The heart valve according to claim 1 or 2 **characterised in that** the thickness ( C ) of the disc ( 2 ) with the circumferential spline ( 9 ) is within the range of 0.06 - 0.12 of the diameter ( D ) of the disc ( 2 ) of the heart valve ( 1 ).

4. The heart valve according to one of the claims 1-3 **characterised in that** the upper surface ( 4 ) of the disc ( 2 ) is a spherical surface with the same radius of curvature as the lower spherical surface ( 5 ) of the disc ( 2 ), and the working surface of the upper holder ( 7 ) is tangent to the curvature of the upper spherical surface ( 4 ) of the disc ( 2 ) in the point ( K ), where the upper holder ( 7 ) touches the upper surface ( 4 ) of the disc ( 2 ) of the heart valve ( 1 ), in each position of the disc ( 2 ) of the heart valve ( 1 ).

5. The heart valve according to one of the claims 1-4 **characterised in that** the lower holders ( 7 ), the upper holders ( 6 ) and the limiter ( 11 ) of the extreme position of the disc ( 2 ) of the heart valve ( 1 ) are situated inside the clearance ( 12 ) of the ring ( 3 ) of the heart valve ( 1 ).

6. The heart valve according to one of the claims 1-5 **characterised in that** the length of each upper holder ( 6 ) and each lower holder ( 7 ) and the limiter ( 11 ) of the extreme position of the disc ( 2 ) of the heart valve ( 1 ) is less than 0.2 of the diameter ( D ) of the disc ( 2 ) of the heart valve ( 1 ).

## Patentansprüche

1. Herzklappe mit einer beweglichen Rundscheibe ( 2 ), die in einen Ring ( 3 ) der Klappe ( 1 ) mittels zwei Halterpaare ( 6, 7 ) eingesetzt ist, die symmetrisch im Verhältnis zur Symmetriefläche ( P ) des Rings ( 3 ) der Klappe (1) angeordnet sind, wobei jedes Halterpaar ( 6, 7 ) einen unteren Halter ( 6 ) und einen oberen Halter ( 7 ) aufweist, und die Rundscheibe ( 2 ) in der geschlossenen Stellung der Klappe ( 1 ) von einem zusätzlichen Vorsprung (11) auf der inneren Fläche des Rings ( 3 ) der Klappe ( 1 ) abgestützt wird, welcher einen Begrenzer der äußersten Lage der Rundscheibe ( 2 ) der Klappe ( 1 ) darstellt und in der Symmetriefläche ( P ) der Klappe ( 1 ) angeordnet ist, wobei die obere Fläche ( 4 ) der Rundscheibe ( 2 ) der Klappe ( 1 ) eine konvexe Fläche und die untere Fläche ( 5 ) der Rundscheibe ( 2 ) der Klappe ( 1 ) eine konkave Fläche ist, **dadurch gekennzeichnet, dass** die unteren Halter ( 6 ), die die Rundscheibe ( 2 ) der Klappe ( 1 ) abstützen, mit einer sphärischen Fläche ( 8 ) abschließen und derartig angeordnet sind, dass die Mittelpunkte ( Z ) der sphärischen Flächen ( 8 ) der unteren Halter ( 6 ) im Grundriss auf die zur Längsachse ( 0 ) der Klappe ( 1 ) senkrechte Fläche, sowie der Punkt ( X ), welcher vom Grundriss der durch die Symmetriefläche ( P ) der Klappe ( 1 ) bestimmten Geradlinie und Umriss der zylindrischen Innenfläche des Rings ( 3 ) der Klappe ( 1 ) festgelegt ist, ein gleichschenkeliges Dreieck ( T ) bilden, dessen Schenkel ( S ) vom Punkt ( X ) zu den Grundrissen der Mittelpunkte ( Z ) der sphärischen Flächen ( 8 ) verlaufen und den Winkel ( A ) zwischen den Schenkeln ( S ) des gegenständlichen, gleichschenkeligen Dreiecks ( T ) aus dem Bereich über 53° und unter 60° bilden, jedoch die Rundscheibe ( 2 ) der Klappe ( 1 ) einen Randvorsprung ( 9 ) um deren Umfang herum aufweist, dessen Innenwand ( 10 ), mit der sich die Rundscheibe ( 2 ) auf sphärischen Flächen ( 8 ) der unteren Halter ( 6 ) stützt, unterm Winkel ( B ) zur Symmetrieachse ( 0' ) der Rundscheibe ( 2 ) der Klappe ( 1 ) verläuft, wobei der Maß des Winkels ( B ) sich im Bereich von 0,5 bis 0,62 des Winkels ( A ) enthält.

2. Herzklappe nach Anspruch 1, **dadurch gekennzeichnet, dass** die Distanz ( G ) in dem zusammengebauten Zustand der Rundscheibe ( 2 ) der Klappe ( 1 ) zwischen dem oberen Halter ( 7 ) und dem unteren Halter ( 6 ) im jeden Halterpaar in dem Bereich von 0,048 bis 0,1 des Durchmessers ( D ) der Rundscheibe ( 2 ) der Klappe ( 1 ) liegt.

3. Herzklappe nach Anspruch 1 oder 2, **dadurch gekennzeichnet, dass** die Dicke ( C ) der Rundscheibe ( 2 ) samt dem Randvorsprung ( 9 ) um den Umfang herum in dem Bereich von 0,06 bis 0,12 des Durchmessers ( D ) der Rundscheibe ( 2 ) der Klappe ( 1 ) beinhaltet ist.

4. Herzklappe nach einem der Ansprüche 1 - 3, **dadurch gekennzeichnet, dass** die obere Fläche ( 4 ) der Rundscheibe ( 2 ) eine sphärische Fläche von dem gleichem Krümmungsradius wie die untere sphärische Fläche ( 5 ) der Rundscheibe ( 2 ) ist, die aktive Fläche des oberen Halters ( 7 ) dagegen tangential zu der Krümmung der unteren sphärischen Fläche ( 4 ) der Rundscheibe ( 2 ) im Punkt ( K ) liegt, in dem der obere Halter ( 7 ) die obere Fläche ( 4 ) der Rundscheibe ( 2 ) der Klappe ( 1 ) in jeder Lage der Rundscheibe ( 2 ) der Klappe ( 1 ) beführt.

5. Herzklappe nach einem der Ansprüche 1 - 4, **dadurch gekennzeichnet, dass** die unteren Halter ( 7 ), die oberen Halter ( 6 ), sowie der Begrenzer ( 11 ) der extremen Lage der der Rundscheibe ( 2 ) der Klappe ( 1 ) innerhalb des lichten Durchmessers ( 12 ) des Rings ( 3 ) der Klappe ( 1 ) lokalisiert sind.

6. Herzklappe nach einem der Ansprüche 1 - 5, **gekennzeichnet dadurch**, dass die Länge von jeden der oberen Haltern ( 6 ) und der unteren Haltern ( 7 ) sowie des Begrenzers ( 11 ) der extremen Lage der der Rundscheibe ( 2 ) der Klappe ( 1 ) kleiner als 0,2 des Durchmessers ( D ) der Rundscheibe ( 2 ) der Klappe ( 1 ) ist.

## Revendications

1. Valve cardiaque comprenant un disque mobile ( 2 ) intégré dans l'anneau ( 3 ) de la valve ( 1 ) au moyen de deux paires de supports ( 6, 7 ), disposés symétriquement par rapport au plan de symétrie ( P ) de l'anneau ( 3 ) de la valve ( 1 ), dans lequel chaque paire de supports ( 6, 7 ) comprend un support inférieur ( 6 ) et un support supérieur ( 7 ), et le disque ( 2 ) dans la position fermée de la valve ( 1 ) est soutenu par une projection supplémentaire ( 11 ) sur la surface intérieure de l'anneau ( 3 ) de la valve ( 1 ) servant de limiteur pour la position maximale du disque ( 2 ) de la valve ( 1 ) situé dans le plan de symétrie ( P ) de la valve ( 1 ), cependant la surface supérieure ( 4 ) du disque ( 2 ) de la valve ( 1 ) est une surface convexe, et la surface inférieure ( 5 ) du disque ( 2 ) de la valve ( 1 ) est une surface concave, **se caractérisant par** le fait, que les supports inférieurs ( 6 ) maintenant le disque ( 2 ) de la valve ( 1 ) se terminent par une surface sphérique ( 8 ) et sont disposés de telle manière que la projection sur un plan perpendiculaire à l'axe longitudinal ( 0 ) de la valve ( 1 ), des centres ( Z ) des surfaces sphériques ( 8 ) des supports ( 6 ) et du point ( X ) déterminé par une ligne droite définie par le plan de symétrie ( P ) de la valve ( 1 ) et le contour de la surface cylindrique intérieure de l'anneau ( 3 ) de la valve ( 1 ) forme un triangle isocèle ( T ) avec des côtés ( S) s'étendant du point ( Z ) jusqu'aux projections des centres ( Z ) des surfaces sphériques ( 8 ), avec un angle ( A) entre les côtés ( S ) du triangle isocèle ( T ) compris entre plus de 53° et en dessous de 60°, cependant le disque (2) de la valve ( 1 ) autour de son pourtour possède une saillie périphérique ( 9 ), dont la paroi intérieure ( 10 ), de laquelle le disque ( 2 ) s'appuie sur les surfaces sphériques ( 8 ) des supports inférieurs ( 6 ), est inclinée d'un angle ( B ) par rapport à l'axe de symétrie ( 0') du disque ( 2 ) de la valve ( 1 ), où la valeur de l'angle ( B ) est comprise entre 0,5 et 0,62 de l'angle ( A ).

2. La valve selon la restriction 1, se caractérise en ceci, que la distance ( G ) à l'état monté du disque ( 2 ) de la valve ( 1 ) entre le support supérieur ( 7 ) et le support inférieur ( 6 ) pour chaque paire de supports est comprise dans un intervalle entre 0,048 - 0,1 du diamètre ( D ) du disque ( 2 ) de la valve ( 1 ).

3. La valve selon la restriction 1 ou 2, se caractérise en ceci, que l'épaisseur ( C ) du disque ( 2 ) ainsi que la saillie circonférentielle ( 9 ) est comprise dans un intervalle entre 0,06 - 0,12 du diamètre ( D ) du disque ( 2 ) de la valve ( 1).

4. La valve selon l'une des restrictions 1 - 3, se caractérise en ceci, que le support supérieur ( 4 ) du disque ( 2 ) est une surface sphérique avec un rayon uniforme de courbure comme la surface inférieure sphérique ( 5 ) du disque ( 2 ), et la surface de travail du support supérieur ( 7 ) est disposée tangentiellement à la courbure de la surface sphérique supérieure ( 4 ) du disque ( 2 ) au point ( K ), dans lequel le support supérieur ( 7 ) est en contact avec la surface supérieure ( 4 ) du disque ( 2 ) de la valve ( 1 ), pour chaque position du disque ( 2 ) de la valve ( 1 ).

5. La valve selon l'une des restrictions 1 - 4, se caractérise en ceci, que les supports inférieurs ( 7 ), les supports supérieurs ( 6 ) ainsi que le limiteur ( 11 ) de la position maximale du disque ( 2 ) de la valve ( 1 ) sont localisés à l'intérieur du passage ( 12 ) de l'anneau ( 3 ) de la valve ( 1 ).

6. La valve selon l'une des restrictions 1 - 5, se caractérise en ceci, que la longueur de chacun des supports supérieurs ( 6 ) et des supports inférieurs ( 7 ) ainsi que du limiteur ( 11 ) de la position maximale du disque ( 2 ) de la valve ( 1 ) est inférieur à 0,2 du diamètre ( D ) du disque ( 2 ) de la valve ( 1 ).
